Europäisches Patentamt

European Patent Office

Office européen des brevets

⑩

(11) Publication number: **0 170 162**
**B1**

(12) **EUROPEAN PATENT SPECIFICATION**

(45) Date of publication of patent specification: **16.05.90**

(21) Application number: **85108983.9**

(22) Date of filing: **18.07.85**

(51) Int. Cl.⁵: **A 61 K 39/10, C 12 P 21/00,**
**A 61 K 35/74, A 61 K 37/02**

(54) Method for the purification of leukocytosis-promoting factor haemagglutinin.

(30) Priority: **19.07.84 JP 150945/84**
**22.08.84 JP 175710/84**
**10.09.84 JP 190244/84**

(43) Date of publication of application:
**05.02.86 Bulletin 86/06**

(45) Publication of the grant of the patent:
**16.05.90 Bulletin 90/20**

(84) Designated Contracting States:
**AT BE CH DE FR GB IT LI NL SE**

(56) References cited:
**EP-A-0 159 003**
**GB-A-2 015 531**
**US-A-4 247 452**

(73) Proprietor: **Juridical Foundation The Chemo-**
**Sero-Therapeutic Research Institute**
**668, Okubo Shimizu-machi**
**Kumamoto-shi Kumamoto-ken (JP)**

(72) Inventor: **Ginnaga, Akihiro**
**3-9-12, Hakenomiya**
**Kumamoto-shi Kumamoto-ken (JP)**
Inventor: **Nishihara, Tsukasa c/o Juridical**
**Foundation**
**The Chemo-Sero-Therapeutic Res. Inst. 668,**
**Okubo**
**Shimizu-machi Kumamoto-shi Kumamoto-ken**
**(JP)**
Inventor: **Kawahara, Tetsuo**
**402, Hokamaki Ozu-machi**
**Kikuchi-gun Kumamoto-ken (JP)**
Inventor: **Susumi, Sadao**
**1-12-13, Kusunoki**
**Kumamoto-shi Kumamoto-ken (JP)**
Inventor: **Mizokami, Hiroshi**
**1647-151, Oaza-Kikudomi Koshi-machi**
**Kikuchi-gun Kumamoto-ken (JP)**
Inventor: **Sakoh, Mitsuo**
**119-5, Kaminogo-machi**
**Kumamoto-shi Kumamoto-ken (JP)**

**EP 0 170 162 B1**

(74) Representative: **Vossius & Partner**
**Siebertstrasse 4 P.O. Box 86 07 67**
**D-8000 München 86 (DE)**

## Description

The present invention relates to a method for the purification of LPF—HA (Leukocytosis-promoting Factor Hemagglutinin). More particularly, it relates to a method for producing LPF—HA in a high yeild and high purity by contacting an LPF—HA-containing solution obtained from culture media of *Bordetella pertussis* with a cellulose sulfate gel, a crosslinked polysaccharide sulfate gel, or a polysaccharide gel chemically bound to a dextran sulfate, thereby adsorbing LPF—HA on the gel, and then eluting LPF—HA from the gel.

LPF—HA is produced by *B. pertussis* phase I and phase II strains. It is not produced by *B. pertussis* phase III strain having no virulence or *Bordetella parapertussis, Bordetella bronchiseptica*. The LPF—HA is also designated as *B. pertussis* toxin and is a protein having various physiological activities. The main physiological activities are a leukocytosis-promoting activity, an insulin secretion-enhancing activity, a histamine-sensitizing and a hemagglutinating activity.

Particularly, because of the insulin secretion-enhancing activity, it is noticed that the LPF—HA may be useful for the treatment of diabetes.

Separately from the above physiological activities, it has recently been noticed that LPF—HA shows an important function in the prophylaxis of infection of *B. pertussis* and infectious disease thereof and hence is useful as an antigen for prophylaxis of infection of *B. pertussis* [cf. Pittman, M.; Review of Infectious Diseases, *1*, 401—409 (1979), and Sato, Y. et al., Seminars in Infectious Diseases IV, Bacterial Vaccines, 380—385 (1982)].

Thus, it has been desired to develop an improved and simple method for the separation and purification of LPF—HA in large quantities on an industrial scale, for the purpose of studying the physiological activities of LPF—HA, the production of a pharmaceutical composition and the production of a pertussis vaccine having less side effects.

According to known methods, the separation and purification of LPF—HA is carried out by salting out a culture medium of *B. pertussis* with ammonium sulfate, extracting and dialyzing, and then subjecting the thus obtained material to ion exchange chromatography, gel filtration [cf. Arai, H., Biochimica et Biophysica Acta, *444*, 765 (1976)] or to sucrose concentration gradient centrifuguation [cf. Sato, Y., Infect Immun., *6*, 897—704 (1972)]. According to such known methods, however, it is difficult to obtain highly purified LPF—HA, and its yield is very low.

In order to obtain the desired highly pure LPF—HA in a comparatively large amount, it is also proposed that the supernatant of culture media of *B. pertussis* is passed through a column packed with hydroxyapatite to adsorb LPF—HA thereon, followed by washing, eluting and then subjecting to affinity chromatography with concanavalin A-Sepharose (Con A-Sepharose, manufactured by Pharmacia ) [cf. Yajima, M. et al., J. Biochem,, *83*, 295—303 (1978)]. However, the affinity chromatography using concanavalin A as a ligand not only has an affinity with LPF—HA but also can adsorb saccharides, glycolipids and also other glycoproteins, and hence, it adsorbs other pertussis cell components such as F—HA (Filamentous Hemagglutinin) and cell membrane components, which results in difficulties in the isolation of the desired highly pure LPF—HA. Thus, it is not suitable as an affinity chromatography for LPF—HA.

Since it recently has been found that human haptoglobin binds specifically to LPF—HA, it has been tried to purify LPF—HA by an affinity chromatography using as a ligand the human haptoglobin instead of the above mentioned concanavalin [cf. Iron, L. et al., Biochimica et Biophysica Acta, *580*, 175—185 (1979), and Cowell, J et al., Seminars in Infectious Diseases IV, Bacterial Vaccines, 371—379 (1982)]. However, a new problem arises from the use of human haptoglobin as a ligand, i.e. it is necessary to determine the absence of hepatitis virus,

That is, since the human haptoglobin is collected from human blood, it may be contaminated with hepatitis virus and further other unknown infectious factors. This problem is also included in case of using other animal blood. Unfortunately, however, there is no method for surely checking the contamination with hepatitis virus, etc. It is also known that the hapatitis virus etc. can be inactivated by heating it at 60°C for 10 to 15 hours. It has been found by the present inventors that when haptoglobin is subjected to such a heat treatment, it looses almost the whole affinity to LPF—HA and hence can not exhibit the desired effect when used in the affinity chromatography.

Moreover, in case of the purification using hydroxyapatite as mentioned above, since the hydroxyapatite is very expensive, this method is not suitable for the purifiation of LPF—HA at low costs and on an industrial scale, either.

The technical problem underlying the present invention, therefore, is to provide an improved method for the separation and purification of LPF—HA on an industrial scale and to provide LPF—HA which is biologically active and is useful in the medical field. Another object of the invention is to provide a method of producing a highly purified LPF—HA which is useful for the preparation of a *B. pertussis* vaccine.

Thus this method for the purification of LPF—HA produced by Bordetella pertussis comprises the steps of contacting an LPF—HA-containing solution at a temperature of 0° to 30°C with a cellulose sulfate gel, a crosslinked polysaccharide sulfate gel or a polysaccharide gel chemically bound to a dextran sulfate, which gel is previously equilibrated with a buffer having a pH of 5.0 to 9.0 and a specific conductivity of 0.5 to 5.0 ms/cm, whereby LPF—HA is not adsorbed by F—HA (Filamentous hemagglutinin) is adsorbed, and eluting LPF—HA from the gel with a buffer having a specific conductivity of 5.0 to 100.0 ms/cm.

The starting culture media of *B. pertussis* include culture media obtained by culturing *B. pertussis* phase I strain in a conventional liquid medium, such as Cohen-Wheeler medium or Stainer-Scholte medium, in a usual manner, such as stationary culture, shaking culture, or spinner culture (this is synonym with shaking culture, aeration culture, and aeration spinner culture). The culture media are used in the method of the present invention after being subjected to centrifugation to remove the cells, or after fracturing the cells followed by centrifugation, or after partial purification.

The sulfuric acid ester of cellulose used as the cellulose sulfate gel in the method of the present invention is obtained by sulfating a cellulose, preferably a crystalline cellulose or cellulose having crystalline area and non-crystalline area. The sulfuric acid ester of cellulose thus obtained retains well the original shape (preferably spherical shape) of the starting material, is insoluble in an aqueous medium, and has excellent physical stability, and hence, is suitable as a gel for chromatography. These starting celluloses are commercially available, for example, Cellulofine GC—15, GH—25, GC—100 or GC—200 (manufactured by Chisso Corp., Japan), and Abicel (manufactured by Asahi Chemical, Japan). The sulfation of the cellulose can be carried out by a conventional method, for example, by treating a gel of cellulose with chlorosulfonic acid, anhydrous sulfuric acid, or another sulfating agent in an organic solvent (e.g. pyridine).

The sulfuric acid ester of a crosslinked polysaccharide includes a sulfuric acid ester of polysaccharides, such as dextran, celluloses, agarose, which is crosslinked with a crosslinking agent, such as epichlorohydrin, dichlorohydrin, dibromohydrin, ethylene glycol bis-epoxypropyl ether. The crosslinked polysaccharides are commercially available, for example, crosslinked dextrans such as Sephades G—10, G—25, G—50 and G—100 (manuactured by Pharmacia, Sweden), crosslinked agaroses such as Sepharose CL—2B, CL—4B and CL—6B (manufactured by Pharmacia, Sweden), and crosslinked celluloses such as Cellulofine GCL—25, GCL—90 (manufactured by Chisso Corp., Japan). The sulfation of the crosslinked polysaccharide can be carried out by a conventional method, for example, by treating a gel of the crosslinked polysaccharide with chlorosulfonic acid, anhydrous sulfuric acid, or another sulfating agent in an organic solvent (e.g. pyridine).

The polysaccharide gel chemically bound to a dextran sulfate is produced by chemically binding a dextran sulfate to a polysaccharide gel derivative. Various products of the dextran sulfate are commercially available, among which the products used usually for biological purposes are preferably used. The polysaccharide gel derivative includes gel derivatives which are prepared by subjecting a polysaccharide (e.g. agarose, dextran, cellulose, etc.) to conventional treatments for giving properties suitable for using as a carrier for chromatography, such as crystallization purification treatment, three-dimensional crosslinking, molding, etc. These products are also commercially available and include, for example, an agarose gel such as Sepharose (manufactured by Pharmacia, Sweden), a dextra gel such as Sephadex (manufactured by Pharmacia, Sweden), a cellulose gel such as Abicel (manufactured by Asahi Chemical, Japan). The chemical linking of the dextran sulfate and the polysaccharide can be done by various methods, for example, buy the method of Andersone et al., using cyanogembromide (cf. Japanese Patent First Publication No. 114018/1977), or a method using cyanogembromide and also lysine (as a spacer) [cf. Bryan M. Turner et al.; Biochemica et Biochimica at Biophysica Acta, *659*, 7—14 (1981)]. One product of dextran sulfate — agarose gel is already on the market, for example; dextran sulfate — Sepharose CL 4B (manufactured by Pharmacia, Sweden).

The isolation and purification of LPF—HA from a culture of *B. pertussis* with these gels is carried out in the following manner.

The starting LPF—HA-containing solution may be prepared by centrifuging a culture of *B. pertussis,* diluting the supernatant with distilled water or a buffer so as to obtain a specific conductivity of 0.1 to 5.0 mS/cm, and then subjecting the solution to the adsorption treatment. However, since the supernatant usually contains F—HA (Filamentous Hemagglutinin) which has also affinity to the cellulose sulfate gel and other gels, the supernatant may be subjected to chromatography with a cellulose sulfate gel or another gel which adsorbs F—HA under such conditions that LPF—HA is not adsorbed but F—HA is adsorbed (wherein the starting solution regulated to a specific conductivity of 5.0—25.0 mS/cm and pH 5—9 is passed through a column packed with a cellulose sulfate gel or another gel, which is equilibrated with a buffer of a specific conductivity of 5.0—2.50 mS/cm and pH 5—9), and then the fraction passed through the column which does not contain F—HA and contains a large amount of LPF—HA is subjected to the adsorption treatment.

The purification treatment comprises adsorption of LPF—HA onto e.g. the cellulose sulfate gel. The washing of the gel containing the LPF—HA and elution of the LPF—HA can be carried out by a conventional operation such as a batch method or a column method. The column method is more advantageous since it is a simpler procedure. In case of the column method, the cellulose sulfate gel or other gels are packed in a column, and it is previously equilibrated by passing through an appropriate buffer having a specific conductivity of 0.5 to 5.0 mS/cm and a pH of about 5.0 to 9.0, for example of 0.02 M McIlvaine's buffer (pH 5.2), and then it is used for the adsorption of LPF—HA.

In the adsorption, the LPF—HA-containing solution is usually regulated to a pH of 5.0 to 9.0 and a specific conductivity of 0.5 to 5.0 mS/cm, and then passed through the column packed with the cellulose sulfate gel or other gel to adsorb LPF—HA. Thereafter, the column is washed with the same buffer as used for the above equilibration, by which contaminated materials are washed out.

The elution of LPF—HA is usually carried out by passing through an appropriate buffer having a pH of 5.0 to 9.0 and a specific conductivity of 5.0 mS/cm or more, usually 5.0 to 100 mS/cm, preferably by stepwise elution or salt concentration gradient elution. That is, when a diluted supernatant obtained by centrifugation of a culture of B. pertussis is used as the starting material, F—HA is also adsorbed together with LPF—HA in the above adsorption condition, and hence, it is necessary to elute LPF—HA under such conditions that can elute LPF—HA but not F—HA. This is done as follows. Firstly, an appropriate buffer having a pH of 5 to 9 and a specific conductivity of 5 to 100 mS/cm, preferably 50 to 60 mS/cm (for example, a 0.7 M sodium chloride-added 0.02 M McIlvaine's buffer) is passed through the column, by which a fraction containing LPF—HA is recovered.

Thereafter, a buffer having a specific conductivity of larger than that of the above buffer for elution (e.g. a specific conductivity of 100 to 300 mS/cm) is passed through, by which F—HA and other impurities are eluted out, followed by equilibrating the cellulose sulfate or other gel in order to re-use the gel.

The most preferable elution is carried out by a salt concentration gradient elution method. In case of using an LPF—HA-containing solution, from which F—HA is previously removed, the elution is carried out by using a buffer having such a salt concentration gradient as a specific conductivity from 0.5 to 300 mS/cm (for example, a 0.02 M McIlvaine's buffer (pH 5.2) having a sodium chloride concentration gradient from 0 to 4.0 M) to obtain a LPF—HA-containing fraction, by which a highly purified LPF—HA can be obtained.

According to the purification method of the present invention, the purification degree of LPF—HA becomes several ten folds and further the recovery rate of LPF—HA reaches to from more than 90% to almost 100%. Besides the purified LPF—HA has a high specific activity of $0.8—0.9 \times 10^5$ LPF—Hp-ELISA unit/mg protein, and further forms a single band in a polyacrylamide disc electrophoresis analysis (pH 4.5), which means that B. pertussis endotoxin is almost completely removed.

Thus, according to the above purification method, the desired LPF—HA can be isolated from the starting culture of B. pertussis in a high yield and high purity in a very simple operation, and the chromatography adsorbent can be prepared with low cost and also can be re-used repeatedly without deterioration. Hence, from an economical point of view, the method is excellent. Accordingly, the purification method of the present invention is suitable as an industrial method for the production of a highly purified LPF—HA. If necessary, the purification may be combined with conventional purification methods, such as sucrose density gradient ultracentrifugation, ion exchange chromatography, etc., by which an even better product can be obtained.

The purified LPF—HA obtained by the present invention is very pure and does not contain other proteins, lipids, saccharides, etc. Furthermore, endotoxins are almost completely removed. Hence, it can be used in various reagents utilizing the biological activity, for the preparation of pharmaceutical compositions and also for the preparation of a B. pertussis vaccine.

The present invention is illustrated by the following Preparations and Examples, but should not be construed to be limited thereto.

Preparation 1

To pyridine (600 ml) dropwise chloro-sulfonic acid (117 g) is added at below 0°C. After the addition, the mixture is heated to 65—70°C. To the mixture crystalline cellulose gel (Cellulofine GC—15, manufactured by Chisso Corp.) (80 g) is added and the mixture is stirred at 65—70°C for 3 hours. After the reaction, the reaction mixture is cooled and neutralized with 10% aqueous sodium hydroxide. The gel thus obtained is separated by filtration and washed well with 0.01 M phosphate buffer-aqueous sodium chloride mixture to give a cellulose sulfate gel.

Preparation 2

To pyridine (600 ml) dropwise chloro-sulfonic acid (117 g) is added at below 0°C. After the addition, the mixture is heated to 65—70°C. To the mixture crystalline cellulose (Abicel for chromatography, manufactured by Asahi Chemical, Japan) (80 g), is added and the mixture is stirred at 65—70°C for 4 hours. After the reaction, the reaction mixture is cooled and neutralized with 10% aqueous sodium hydroxide. The gel thus obtained is separated by filtration and washed well with 0.01 M phosphate buffer-aqueous sodium chloride mixture to give a cellulose sulfate gel.

Preparation 3

To pyridine (200 ml) dropwise chloro-sulfonic acid (11 ml) is added at below 0°C. After the addition, the mixture is heated to 65—70°C. To the mixture epichlorohydrin-crosslinked dextran (Sephadex G—50, manufactured by Pharmacia, Sweden) (7.5 g) is added, and the mixture is stirred at 65—70°C for 4 hours. After the reaction, the reaction mixture is cooled and neutralized with aqueous sodium hydroxide. The gel thus obtained is separated by filtration and washed well with 0.01 M phosphate buffered saline solution to give a crosslinked dextran sulfate.

Preparation 4

To a mixture (210 ml) of pyridine and chlorosulfonic acid prepared in the same manner as described in Preparation 3 crosslinked cellulose (Cellulofine GCL—25, manufactured by Chisso Corp., Japan) (7.5 g) is added and the mixture is stirred at 65—70°C for 4 hours. After the reaction, the reaction mixture is cooled and then neutralized with aqueous sodium hydroxide. The gel thus obtained is separated by filtration and washed well with 0.01 M phosphate buffered saline solution to give a crosslinked cellulose sulfate (7.2 g).

Preparation 5

To a mixture (210 ml) of pyridine an chlorosulfonic acid prepared in the same manner as described in Preparation 3 crosslinked agarose (Sepharose CL—6B, manufactured by Pharmacia, Sweden) containing pyridine is added, and the mixture is stirred at 65—70°C for 4 hours. After the reaction, the reaction mixture is cooled and neutralized with aqueous sodium hydroxide. The gel thus obtained is separated by filtration and washed well with 0.01 M phosphate buffered saline solution to give a crosslinked agarose sulfate (23 ml).

Preparation 6

Sodium dextran sulfate (5 g) in 0.5 M aqueous sodium carbonate (200 ml) is dissolved, and thereto Sepharose CL—4B (agarose gel, manufactured by Pharmacia, Sweden) (20 ml) which is equilibrated by 0.5 M aqueous sodium carbonate is added, and the mixture is gently stirred. To the mixture with stirring a solution of cyanogen bromide (10 g) in distilled water (100 ml) is added. The mixture is maintained for 15 minutes while keeping at pH 11 by adding 5 M aqueous sodium hydroxide. Thereafter, the mixture is stirred at room temperature for 17 hours, while allowing to lower the pH value. After the reaction, the reaction mixture is filtered with a glass filter, and the gel thus obtained is washed well with 0.15 M sodium chloride-added phosphate buffer (pH 7.2) to give dextran sulfate agarose gel (20 ml).

Example 1

The Cellulofine GC—15 sulfate gel obtained in the same manner as described in the above Preparation 1 is packed within a column (40 mmφ × 200 mm), and therethrough is passed distilled water (1.0 liter). The supernatant (500 ml) of a fermenter culture of *B. pertussis* phase I Tohama strain is 10 fold diluted with distilled water and the diluted solution (specific conductivity: about 1.5 mS/cm) is passed through the column. After washing well the column with 0.02 M McIlvaine's buffer (pH 5.2, about 500 ml), the adsorbed material is eluted with 0.02 M sodium chloride-added McIlvaine's buffer (specific conductivity: about 2.0 mS/cm, pH 5.2, 2,000 ml) in a concentration gradient of sodium chloride from 0 to 4.0 M, whereby fractions (each about 20 ml) are collected and then the fraction containing LPF—HA (about 130 ml) is pooled.

The analytical data and experimental data of the starting material and the purified LPF—HA fraction are shown in Table 1.

TABLE 1

| | Samples | |
|---|---|---|
| | Supernatant of culture (starting material) | Fraction of purified LPF—HA |
| Amount of sample (ml) | 500 | 130 |
| Content of LPF—HA (1) (LPEU/ml) | 500 | 1,750 |
| Content of protein (2) (mg/ml) | 0.250 | 0.020 |
| Specific activity of LPF—HA (LPEU/mg protein) | $2 \times 10^3$ | $8.8 \times 10^4$ |
| Recovery rate of LPF—HA (%) | (100) | 84 |
| Pyrogen test in rabbits (3) | 4.5 | 0.9 |

[Notes]: (1) It shows LPF—HA unit which was measured by in vitro test: Hapto-ELISA method (cf. Sato, et al, Symposium on Toxins, Proceeding of the 28th Symposium on Toxins, 141—144 (1981).
(2) It is shown in the protein content when calculated as protein nitrogen determined by the Kjeldahl method × 6.25.
(3) It was done in accordance with the method described in Minimum Requirement of Biological Products, Ministry of Health and Welfare, Japan #287, 1981, wherein the test sample was diluted to a protein content of 6.25 µg/ml. Value given is total increase in body temperature (Sum of three rabbits).

6

## EP 0 170 162 B1

### Example 2

Cellulose sulfate gel obtained in the same manner as described in the above Preparation 1 (each 1 liter) is packed into two columns (A) and (B) (80 mmφ × 200 mm). Column (A) is equilibrated by passing through a 0.2 M sodium-added 0.01 M phosphate buffer (pH 7.2, specific conductivity: 21.0 mS/cm); column (B) is equilibrated with distilled water.

A supernatant (20.0 liters) of a fermenter culture of *B. pertussis* phase I Tohama strain is passed through the above column (A) which is equilibrated with phosphate buffer. The fraction passed through the column (A) is pooled. Besides, the column (A) is washed with 0.2 M sodium chloride-added 0.01 M phosphate buffer (specific conductivity: 21.0 mS/cm), and the fraction (21.0 liters) of wash liquid which contains LPF—HA is also pooled together with the above through fraction. The pooled fractions are diluted with distilled water to regulate the specific conductivity to about 1.5 mS/cm) and are passed through the column (B), which is equilibrated with distilled water. After washing well the column with 0.02 M McIlvaine's buffer (specific conductivity: 2.0 mS/cm, pH 5.2, about 20 liters), the adsorbed material is eluted with 0.02 M McIlvaine's buffer (pH 5.2, 10 liters) in a concentration gradient of sodium chloride from 0 to 4.0 M, whereby fractions (1.1 liter) containing LPF—HA are collected.

The analytical data and experimental data of the starting material and the purified LPF—HA fraction are shown in Table 2.

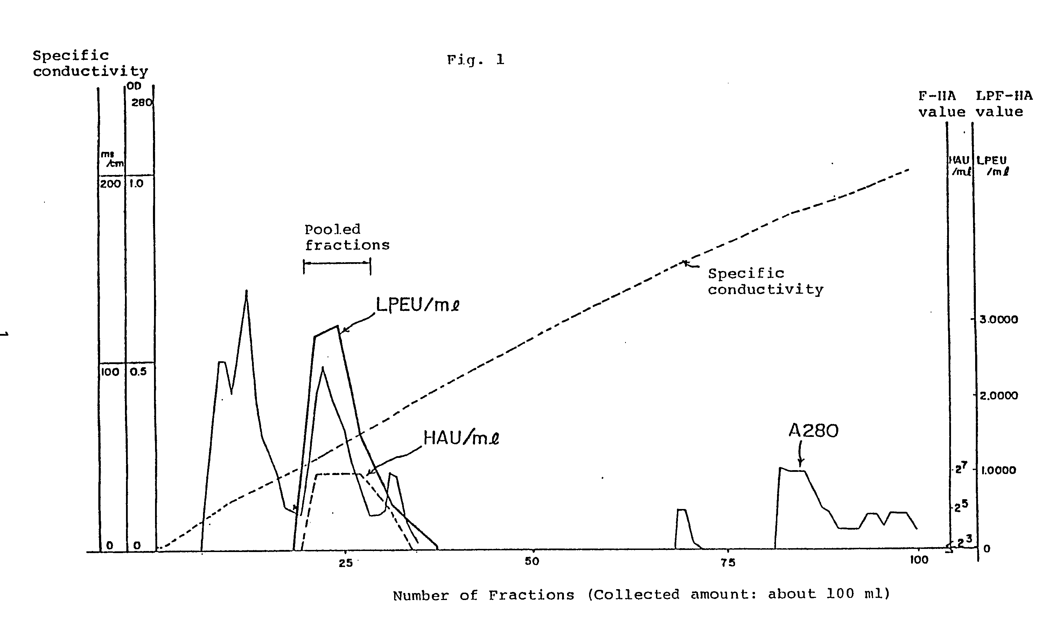

Besides, the chromatogram of the eluted solution from the LPF—HA-adsorbed cellulose sulfate gel is shown in the accompanying Fig. 1. In Fig. 1, the abscissa axis means the number of fractions (amount: about 100 ml), and the ordinate axis means the absorption value at a wave length of 280 nm ($A_{280}$) and the specific conductivity (mS/cm) of the fraction, the LPF—HA content (LPEU/ml) of the fraction which is measured by the hapto-ELISA method and the HA value (HAU/ml) of the fraction which is measured by the agglutination test in chicken [cf. Sato, Y. et. al., Infect. Immun. *7*, 929 (1973)].

### TABLE 2

| | Samples | |
| --- | --- | --- |
| | Supernatant of culture (starting material) | Fraction of purified LPF—HA |
| Amount of sample (ml) | 20,000 | 1,100 |
| Content of LPF—HA (1) (LPEU/ml) | 1,000 | 16,500 |
| Content of protein (2) (mg/ml) | 0.360 | 0.180 |
| Specific activity of LPF—HA (LPEU/mg protein) | $2.8 \times 10^3$ | $9.2 \times 10^4$ |
| Recovery rate of LPF—HA (%) | (100) | 90 |
| Pyrogen test in rabbits (3) | 5.3 | 1.1 |

[Notes]: The notes in (1), (2) and (3) are the same as in the above Table 1.

### Example 3

The crosslinked cellulose sulfate gel (5 ml) obtained in the same manner as described in the above Preparation 4 is packed within a column (40 mmφ × 220 mm), and therethrough is passed distilled water (200 ml). The supernatant (100 ml) of a fermenter culture of *B. pertussis* phase I Tohama strain is 7 fold diluted with distilled water and the diluted solution (specific conductivity: about 3.0 mS/cm) is passed through the column. After washing well the column with 0.02 M McIlvaine's buffer (pH 5.2, about 200 ml), the adsorbed material is eluted with 0.02 M sodium chloride-added McIlvaine's buffer (specific conductivity: about 2.0 mS/cm, pH 5.2, 50 ml) in a concentration gradient of sodium chloride from 0 to 4.0 M, whereby fractions (each about 1 ml) are collected and then the fraction containing LPF—HA (about 6 ml) is pooled.

The analytical data and experimental data of the starting material and the purified LPF—HA fraction are shown in Table 3.

7

TABLE 3

| | Samples | |
| --- | --- | --- |
| | Supernatant of culture (starting material) | Fraction of purified LPF—HA |
| Amount of sample (ml) | 100 | 6 |
| Content of LPF—HA (1) (LPEU/ml) | 500 | 6,400 |
| Content of protein (2) (mg/ml) | 0.250 | 0.073 |
| Specific activity of LPF—HA (LPEU/mg protein) | $2 \times 10^3$ | $8.8 \times 10^4$ |
| Recovery rate of LPF—HA (%) | (100) | 77 |
| Pyrogen test in rabbits (3) | 4.7 | 0.9 |

[Notes]: The notes in (1), (2) and (3) are the same as in the above Table 1.

Example 4

The dextran sulfate agarose gel (5 ml) obtained in the same manner as described in the above Preparation 6 is packed within a column (40 mmφ × 200 mm), and therethrough is passed distilled water (100 ml). The supernatant (100 ml) of a fermenter culture of *B. pertussis* phase I Tohama strain is 8 fold diluted with distilled water and the diluted solution (specific conductivity: about 3.0 mS/cm) is passed through the column. After washing well the column with 0.02 M McIlvaine's buffer (pH 5.2, about 300 ml), the adsorbed material is eluted with 0.02 M sodium chloride-added McIlvaine's buffer (pH 5.2, 100 ml) in a concentration gradient of sodium chloride from 0 to 4.0 M, whereby fractions (each about 2 ml) are collected and then the fraction containing LPF—HA (about 12 ml) is pooled.

The analytical data and experimental data of the starting material and the purified LPF—HA fraction are shown in Table 4.

TABLE 4

| | Samples | |
| --- | --- | --- |
| | Supernatant of culture (starting material) | Fraction of purified LPF—HA |
| Amount of sample (ml) | 100 | 12 |
| Content of LPF—HA (1) (LPEU/ml) | 500 | 3,750 |
| Content of protein (2) (mg/ml) | 0.250 | 0.042 |
| Specific activity of LPF—HA (LPEU/mg protein) | $2 \times 10^3$ | $8.8 \times 10^4$ |
| Recovery rate of LPF—HA (%) | (100) | 90 |
| Pyrogen test in rabbits (3) | 4.7 | 0.9 |

[Notes]: The notes in (1), (2) and (3) are the same as in the above Table 1.

8

**Claims**

1. Method for the purification of LPF—HA (Leukocytosis promoting factor hemagglutinin) produced by *Bordetella pertussis* which comprises contacting an LPF—HA-containing solution at a temperature of 0° to 30°C with a cellulose sulfate gel, a crosslinked polysaccharide sulfate gel or a polysaccharide gel chemically bound to a dextran sulfate, which gel is previously equilibrated with a buffer having a specific pH of 5.0 to 9.0 and a specific conductivity of 0.5 to 5.0 mS/cm, whereby LPF—HA is not adsorbed but F—HA (Filamentous Hemagglobinin) is adsorbed, and eluting LPF-HA from the gel with a buffer having a specific conductivity of 5.0 to 100.0 mS/cm.

2. The method according to claim 1, wherein the LPF—HA-adsorbed gel is washed with a buffer having a specific conductivity of 0.5 to 5.0 mS/cm before the elution.

3. The method according to claim 1, wherein the cellulose sulfate is a sulfuric acid ester of a crystalline cellulose or a cellulose having crystalline areas and non-crystalline areas.

4. The method according to claim 1, wherein the crosslinked polysaccharide sulfate is a crosslinked cellulose sulfate, a crosslinked agaros sulfate, or a crosslinked dextran sulfate.

5. The method according to claim 4, wherein the crosslinked cellulose sulfate is an epichlorohydrin-crosslinked cellulose sulfate.

6. The method according to claim 4, wherein the crosslinked agarose sulfate is an epichlorohydrin-crosslinked agarose sulfate.

7. The method according to claim 4, wherein the crosslinked dextran sulfate is an epichlorohydrin-crosslinked dextran sulfate.

8. The method according to claim 1, wherein the polysaccharide gel chemically bound to a dextran sulfate is a dextran sulfate-agarose gel, a dextran sulfate-dextran gel, or a dextran sulfate-cellulose gel.

9. The method according to claim 1, wherein the LPF—HA-containing solution has been prepared by centrifuging a culture of *B. pertussis*, diluting the supernatant with distilled water or a buffer so as to obtain a specific conductivity of 5.0—25.0 mS/cm and pH 5—9, passing the solution through a column packed with a cellulose sulfate gel or another gel which adsorbs F—HA gel equilibrated with a buffer of a specific conductivity of 5.0—25.0 mS/cm and pH 5—9, and selecting the fraction which is free of F—HA and contains a large amount of LPF—HA for the adsorption treatment.

10. The use of LPF—HA prepared according to any one of claims 1 to 8 for the preparation of a pertussis vaccine.

**Patentansprüche**

1. Verfahren zur Reinigung von LPF—HA (Leukocytosis promoting factor Hemagglutinin), der von *Bordetella pertussis* erzeugt wird, umfassend das Zusammenbringen einer LPF—HA enthaltenden Lösung bei einer Temperatur von 0 bis 30°C mit einem Cellulosesulfatgel, einem vernetzten Polysaccharidsulfatgel oder einem Polysaccharidgel, das chemisch an ein Dextransulfat gebunden ist, wobei das Gel vorher mit einem Puffer mit einem pH-Wert von 5,0 bis 9,0 und einer spezifischen Leitfähigkeit von 0,5 bis 5,0 mS/cm äquilibriert worden ist, wobei LPF—HA nicht adsorbiert wird, jedoch F—HA (Filamentous Hemagglutinin) adsorbiert wird, und Eluierung des LPF—HA aus dem gel mit einem Puffer mit einer spezifischen Leitfähigkeit von 5,0 bis 100,0 mS/cm.

2. Verfahren nach Anspruch 1, wobei das LPF—HA-adsorbierte Gel vor der Eluierung mit einem Puffer mit einer spezifischen Leitfähigkeit von 0,5 bis 5,0 mS/cm gewaschen wird.

3. Verfahren nach Anspruch 1, wobei das Cellulosesulfat ein Schwefelsäureester einer kristallinen Cellulose oder einer Cellulose mit kristallinen Bereichen und nicht-kristallinen Bereichen ist.

4. Verfahren nach Anspruch 1, wobei das vernetzte Polysaccharidsulfat ein vernetztes Cellulosecsulfat, ein vernetztes Agarosesulfat oder ein vernetztes Dextransulfat ist.

5. Verfahren nach Anspruch 4, wobei das vernetzte Cellulosesulfat ein mit Epichlorhydrin vernetztes Cellulosesulfat ist.

6. Verfahren nach Anspruch 4, wobei das vernetzte Agarosesulfat ein mit Epichlorhydrin vernetztes Agarosesulfat ist.

7. Verfahren nach Anspruch 4, wobei das vernetzte Dextransulfat ein mit Epichlorhydrin vernetztes Dextransulfat ist.

8. Verfahren nach Anspruch 1, wobei das chemisch an ein Dextransulfat gebundene Polysaccharidgel ein Dextransulfat-Agarosegel, ein Dextransulfat-Dextrangel oder ein Dextransulfat-Cellulosegel ist.

9. Verfahren nach Anspruch 1, wobei die LPF—HA-enthaltende Lösung hergestellt wurde durch Zentrifugieren einer Kultur von *B. pertussis* Verdünnen des Überstandes mit destilliertem Wasser oder einem Puffer, so daß eine spezifische Leitfähigkeit von 5,0 bis 25,0 mS/cm und ein pH-Wert von 5 bis 9 erhalten wird, Hindurchführen der Lösung durch eine Säule, die mit einem Cellulosesulfatgel oder einem anderen Gel, das F—HA adsorbiert, gepackt ist, welches Gel mit einem Puffer mit einer spezifischen Leitfähigkeit von 5,0 bis 25,0 mS/cm und einem pH-Wert von 5 bis 9 äquilibriert ist, und Auswählen der Fraktion, die frei von F—HA ist und eine große Menge LPF—HA enthält für die Adsorptionsbehandlung.

10. Verwendung des nach einem der Ansprüche 1 bis 8 hergestellten LPF—HA zur Herstellung eines Pertussis-Impfstoffs.

9

**Revendications**

1. Procédé pour la purification de LPF—HA (Leukocytosis-promoting Factor Hemagglutinin) produite par *Bordetella pertussis,* qui comprend le contact d'une solution contenant de la LPF—HA à une température de 0 à 30°C avec un gel de sulfate de cellulose, un gel de sulfate de polysaccharide réticulé ou un gel de polysaccharide lié chimiquement à une sulfate de dextran, lequel gel a été préalablement équilibré avec un tampon ayant un pH de 5,0 à 9,0 et une conductivé spécifique de 0,5 à 5,0 mS/cm, afin que la LPF—HA ne soit pas adsorbée, mais la F—HA (Filamentous Hemagglutinin) soit adsorbée, et l'élution de la LPF—HA du gel avec un tampon ayant une conductivité spécifique de 5,0 à 100,0 mS/cm.

2. Procédé selon la revendication 1, dans lequel le gel ayant adsorbé la LPF—HA est lavé avec un tampon ayant une conductivité spécifique de 0,5 à 5,0 mS/cm avant l'élution.

3. Procédé selon la revendication 1, dans lequel le sulfate de cellulose est un ester d'acide sulfurique d'une cellulose cristalline ou d'une cellulose ayant des régions cristallines et des régions non cristallines.

4. Procédé selon la revendication 1, dans lequel le sulfate de polysaccharide réticulé est un sulfate de cellulose réticulée, un sulfate d'agarose réticulé ou un sulfate de dextran réticulé.

5. Procédé selon la revendication 4, dans lequel le sulfate de cellulose réticulée est un sulfate de cellulose réticulée avec de l'épichlorhydrine.

6. Procédé selon la revendication 4, dans lequel le sulfate d'agarose réticulé est un sulfate d'agarose réticulé avec de l'épichlorhydrine.

7. Procédé selon la revendication 4, dans lequel le sulfate de dextran réticulé est un sulfate de dextran réticulé avec de l'épichlorhydrine.

8. Procédé selon la revendication 1, dans lequel le gel de polysaccharide lié chimiquement à un sulfate de dextran est un gel d'agarose-sulfate de dextran, un gel de dextran-sulfate de dextran ou un gel de cellulose-sulfate de dextran.

9. Procédé selon la revendication 1, dans lequel la solution contenant la LPF—HA a été préparée par centrifugation d'une culture de *B. pertussis,* dilution du surnageant avec de l'eau distillée ou un tampon de façon à obtenir une conductivité spécifique de 5,0 à 25,0 mS/cm et un pH de 5 à 9, passage de la solution à travers une colonne garnie d'une gel de sulfate de cellulose ou d'un autre gel qui adsorbe la F—HA, lequel gel est équilibré avec un tampon ayant une conductivité spécifique de 5,0 à 25,0 mS/cm et ayant un pH de 5 à 9, et sélection de la fraction qui est dépourvue de F—HA et contient une quantité importante de LPF—HA pour le traitement d'adsorption.

10. L'utilisation de la LPF—HA préparée selon l'une quelconque des revendications 1 à 8 pour la préparation d'un vaccin anticoquelucheux.

Fig. 1

Specific
conductivity

F-HA    LPF-HA
value   value

Pooled
fractions

LPEU/mℓ

Specific
conductivity

HAU/mℓ

A280

Number of Fractions (Collected amount: about 100 ml)

EP 0 170 162 B1